# EUROPEAN PATENT APPLICATION

(11) **EP 1 317 900 A1**
(43) Date of publication of application: **11.06.2003**
(21) Application number: 01963546.5
(22) Date of filing: 10.09.2001
(51) Int. Cl.: A61B 3/14

(54) **TO-BE-TESTED EYE PHOTOGRAPHING DEVICE**

(30) Priority: 11.09.2000 JP 2000274281
(71) Applicant: Kowa Kabushiki Kaisha, Nagoya-shi, Aichi 460-8625 (JP)
(72) Inventor: KOBAYASHI, Koji Kowa K. K. Raifusaiensu-jigyobu, Hamamatsu--shi, Shizuoka 431-2103 (JP); SUZUKI, Takayoshi Kowa K. K. Raifusaiensu-jigyobu, Hamamatsu-shi, Shizuoka 431-2103 (JP); IMAMURA, Hiroki Kowa K. K. Raifusaiensu-jigyobu, Hamamastu-shi, Shizuoka 431-2103 (JP); NAGAYAMA, Hiroki Kowa K. K. Raifusaiensu-jigyobu, Aoba-ku, Sendai-shi, Miyagi- 980-0801 (JP)
(74) Representative: Sturt, Clifford Mark
(86) International application number: JP0107821
(87) International publication number: WO02022005

(57) **Abstract**

When a photographing switch (55) is operated, a mirror (26) is moved out of the optical path, a strobe (14) is made to emit a flash of light, and the eye to be examined is photographed by a photographing unit (33). The image of the eye is recorded in image primary recording means (52). In such an arrangement, the image of an eye to be examined can be recorded in image primary recording means provided in the unit of the photographing device. Therefore, the image can be taken independently of the recording preparation state of an external recording device, the timing of flash of the strobe can be set not through an external recording device, and the image of an eye can be reliably and smoothly recorded. Further, since the image recorded in image primary recording means can be recorded in image secondary recording means (60), the same effect of recording the image in an external recording device can be produced if the image secondary recording means is provided to an external recording device.

## Description

### Technical Field

The present invention relates to a device for photographing an eye to be examined, and more particularly to a device for photographing an eye to be examined having a function of photographing an eye, particularly the fundus of an eye to be examined in response to strobe flash.

### Background Art

In the prior art, there are known photographing devices, such as that described in Japanese Patent Publication No. 2927493, in which a strobe emits a flash of light and the fundus of an eye to be examined is photographed by a CCD camera or the like and the image of the eye to be examined is recorded using an external image recording device connected to the photographing device unit.

Since the operation of the photographing device unit is limited by the operating state of the image recording device in prior devices for photographing an eye to be examined, it is possible for photographing errors to occur without any images recorded if the recording preparation is not set and the photographing is initiated. There is also the problem that, since the timing of the strobe flash in the photographing device unit is synchronized with the external recording device, the photographing device unit has to receive a light emission permission signal via the recording device, which limits the types of image recording devices that can be connected.

Another problem that has been pointed out with respect the prior art devices is that, upon photographing operations, it is necessary to operate switches on both the eye photographing device unit and the image recording device, which makes the system more complex to handle and can cause unnecessary images to be transmitted to the recording device and recorded due to errors made in the recording preparation settings of the external recording device.

An object of the invention is to resolve such problems and provide a device or system for photographing an eye to be examined that can reliably and smoothly photograph an eye to be examined.

### Disclosure of Invention

To resolve the above problems, the present invention provides a device for photographing an eye to be examined in which a strobe is made to emit a flash of light to photograph an eye to be examined, comprising a means for activating the strobe, a photographing unit for photographing an eye to be examined illuminated by strobe flash, and image primary recording means for primarily recording an image of the eye to be examined photographed by the photographing unit, wherein an image recorded in the image primary recording means can be recorded in image secondary recording means that is connectable to the image primary recording means.

In such an arrangement, the image of an eye to be examined can be recorded in the image primary recording means provided in a unit of the eye photographing device. Therefore, the image can be taken independently of the recording preparation state of an external recording device, and the timing of the strobe flash can be set not through an external recording device. This allows the image of an eye to be reliably and smoothly recorded.

Also, since the image primary recording means can be connected to the image secondary recording means and an image recorded in the image primary recording means can be recorded in the image secondary recording means, the same effect of recording the image in an external recording device can be produced if the image secondary recording means is provided in an external recording device.

It is preferable that after the image of the eye to be examined is recorded in the image primary recording means, the image is automatically recorded in the image secondary recording means after a prescribed time has elapsed. However, instead of this being done automatically, the image of the eye recorded in the image primary recording means can be recorded manually in the image secondary recording means in response to the operation of a recording switch. In this case, the examiner can first perform a diagnosis using the eye fundus image recorded in the unit of the eye photographing device and then transfer to the image secondary recording means only those images that need to be permanently preserved in the examiner's judgment. Therefore, this makes it possible to operate the system efficiently by conserving the recording capacity of the image secondary recording means.

After an image of the eye to be examined has been recorded in the image primary recording means, it is also preferable to record the image onto CD-R or CD-RW or the like together with image reading software. In cases in which the image of the eye is recorded on CD-RW, it is possible to view-the eye images by using the image reading software recorded on the CD-RW, and to append information to the images. In such an arrangement, images of an eye can be reproduced on a device such as a personal computer, for example, and supplemental information such as diagnostic comments can be appended to the image, expanding the range of image utilization.

### Brief Description of Drawings

Figure 1 is a schematic diagram showing the overall arrangement of a device for photographing an eye to be examined according to the present invention;
Figure 2 is a timing chart showing the photographing and recording timing when the eye is being photographed;
Figure 3 is a schematic diagram showing the overall arrangement of a device for photographing an eye to be examined according to another embodiment of the present invention; and
Figure 4 is a schematic diagram showing the overall arrangement of a device for photographing an eye to be examined according to still another embodiment of the present invention.

### Best Mode for Carrying out the Invention

Details of the present invention will now be described with reference to the embodiments shown in the drawings.

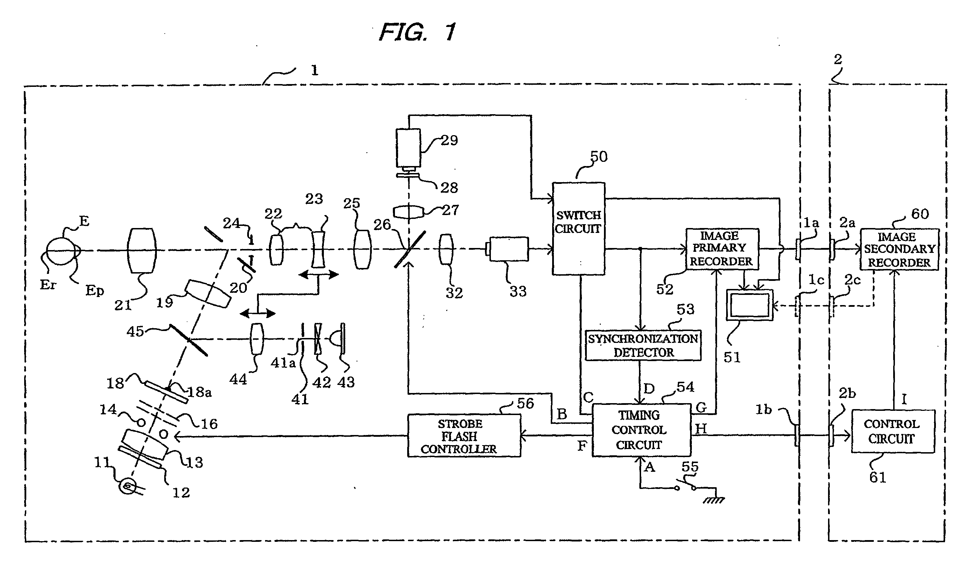

Figure 1 shows an eye photographing device 1 of the present invention and an external recording device 2 connected thereto. The eye photographing device 1 and external recording device 2 constitute a system for photographing an eye to be examined.

The eye photographing device 1 is provided with a lamp 11 that is an observation light source. Light emitted by this lamp passes through an infrared filter 12, a condenser lens 13 and a strobe 14 that is a photographing light source, and illuminates a ring slit 16. The ring slit 16 has a prescribed outside diameter and inside diameter and forms an annular illumination light beam at a position that is substantially conjugate with the pupil Ep of the eye to be examined E.

The light beam from the ring slit 16 passes through a black-point plate 18 that has a black point 18a for removing reflections from an objective lens 21, passes through an infrared half-mirror 45 and relay lens 19 and is reflected by a total-reflection mirror 20 having a center hole. The light beam reflected by the total-reflection mirror 20 is formed into an image at the pupil Ep of the eye E by the objective lens 21, and then falls incident on the eye fundus Er, providing substantially uniform infrared illumination of the eye fundus.

A focusing index 41 having a small center hole 41a is provided for forming a focus dot on the eye fundus for focusing. The focusing index 41 is illuminated.by an infrared LED 43 via an image prism 42. After the light beam passes through a focusing lens 44 it is reflected by an infrared half-mirror 45 and combined with the light beam of the illumination optical system, projecting the focusing index 41 onto the eye fundus Er to form a focus dot thereon.

The focusing lens 44 is mechanically linked with a focusing lens 23 of the photographing optical system so that, when the focusing lens 23 is adjusted for focus into the eye fundus Er to be examined, the focusing index 41 forms an image on the eye fundus Er, that is, the position of the focusing index 41 is approximately conjugate with the position of the eye fundus Er.

Light reflected from the eye fundus Er passes back through the center part of the pupil Ep, the objective lens 21 and the hole in the total-reflection mirror 20, and then through photographing stop 24, focusing lenses 22 and 23 and image-formation lens 25 disposed on the optical path of the photographing optical system, and falls incident on return mirror 26. The light beam from the eye fundus reflected by the return mirror 26 is converged by an image-formation lens 27 onto an infrared photographing unit (CCD camera) 29 sensitive to infrared rays with an infrared filter 28 disposed in front thereof, enabling the eye fundus image to be photographed by the infrared photographing unit 29. The eye fundus image photographed by the infrared photographing unit 29 is displayed via a switch circuit 50 on a liquid-crystal or other such monitor 51 to enable the image to be viewed by the examiner.

An image-formation lens 32 and a visible-light photographing unit 33 sensitive to visible rays are disposed behind the return mirror 26. When the visible-light photographing unit 33 is operated by a photographing switch 55, it is connected to an image primary recording means or recording device 52 comprised of VRAM or flash memory or the like via a switch circuit 50 that is operated by a timing control circuit 54. A vertical synchronization signal is then extracted from the video signal of the visible-light photographing unit 33 by a synchronization detecting circuit 53. The image primary recording means 52 is connected to a monitor 51 to enable images recorded by the image primary recording means 52 to be displayed thereon. When the photographing switch 55 is operated, the timing control circuit 54 is activated. This causes the return mirror 26 to be swung up to retract it from the optical path and a strobe flash control circuit 56 to be operated to cause the strobe 14 to emit light.

The eye photographing device 1 can be connected to the external recording device 2, which has an image secondary recording means or recording device 60 such as a hard disk, MO, DVD or printer, and a control circuit 61. When both the devices 1 and 2 are connected, connectors 1a and 1b of the eye photographing device 1 are respectively connected to the connectors 2a and 2b of the external recording device 2. This establishes the connection of the image primary recording means 52 and image secondary recording means 60 and the connection of the timing control circuit 54 and control circuit 61. Optionally, the image secondary recording means 60 can be connected to the monitor 51 by means of connecter 1c of the eye photographing device 1 and connecter 2c of the external recording device 2.

The operation of the eye photographing device and system thus constituted will now be explained.

During observation of the eye to be examined, the lamp 11 is activated and a beam from which light in the infrared wavelength region has been extracted by the infrared filter 12 passes through the condenser lens 13, the ring slit 16, the black-point plate 18, the infrared half-mirror 45 and the relay lens 19 and is reflected by the total-reflection mirror 20. The beam thus reflected passes through the pupil Ep of the eye to be examined E and falls incident on the eye fundus Er, illuminating the fundus with infrared rays. Light reflected from the fundus Ep passes through the center part of the pupil Ep, the objective lens 21, the hole in the total-reflection mirror 20, the photographing stop 24 and the lenses 22, 23 and 25 and is reflected by the return mirror 26. The reflected light is converged by the image-formation lens 27 onto the infrared photographing unit 29, thus forming an eye fundus image on the infrared photographing unit 29. The switch circuit 50 is now switched to the infrared photographing unit 29, so that the fundus image can be reproduced on the monitor 51 for observation by the examiner.

During the observation, infrared LED 43 is activated at the same time and illuminates the focusing index 41 via the image prism 42. After the infrared beam passes through the hole 41a of the focusing index 41, it is reflected by the infrared half-mirror 45, combined with the illumination beam and projected onto the eye fundus Er as a focus dot. When the focusing lens 23 is adjusted, the focusing lens 44 interlocked therewith is also adjusted. When a clear focus dot is seen, the eye fundus is in focus with respect to the photographing optical system. Thus, with the focus dot showing on the monitor 51, the examiner can focus the focal point on the eye fundus by using the focusing lens 23 to adjust the focal state.

This observation of the eye fundus is conducted with the eye illuminated by infrared rays. This allows the eye to be observed with the pupil in a non-dilated state, reducing the burden on the patient.

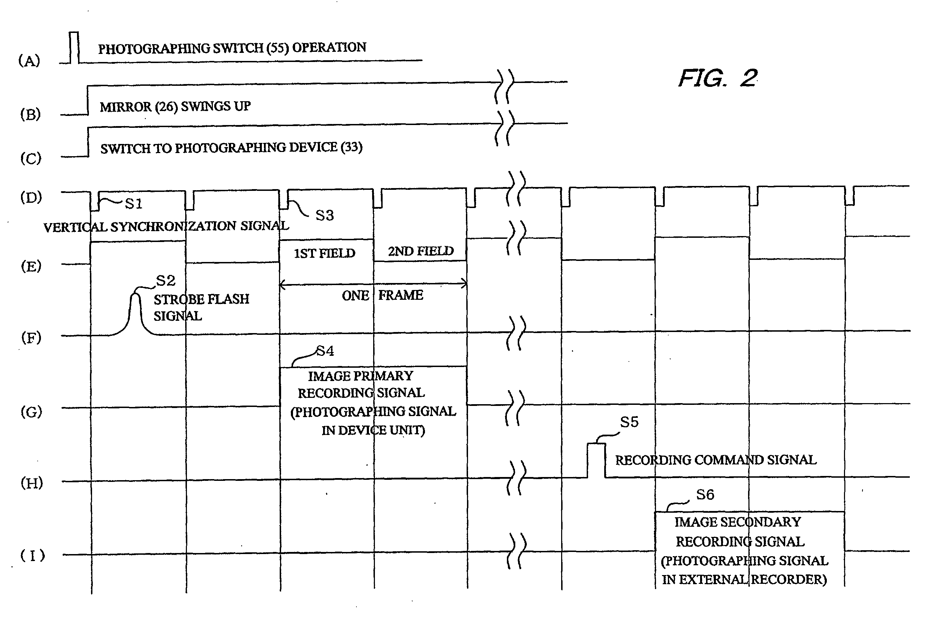

When the focusing has been completed, the photographing switch (shutter) 55 is operated. At this point, the timing control circuit 54 operates to swing the return mirror 26 up out of the optical path and activate the switch circuit 50 to switch the photographing unit from the infrared photographing unit 29 to the visible-light photographing unit 33 at prescribed timings as shown in Figure 2 (A), (B) and (C), and a video signal from the visible-light photographing unit 33 is input to the image primary recording means 52 and to the synchronization detecting circuit 53, which outputs a vertical synchronization signal as shown in Figure 2 (D).

The timing control circuit 54 sends a strobe flash signal S2 as shown in Figure 2 (F) to the strobe flash control circuit 56 in synchronization with the timing of vertical synchronization signal S1. This causes the strobe 14 to flash to illuminate the eye fundus with visible rays and the image to be photographed by the visible-light photographing unit 33. At this time, the timing control circuit 54 outputs to the image primary recording means 52 an image primary recording signal S4 in synchronization with vertical synchronization signal S3 of the first field (odd-numbered field) following the strobe flash signal S2. The period of the image primary recording signal S4 is the length of a first field and second field (even-numbered field), that is, the period of one frame, so that one frame of fundus image is recorded in the image primary recording means 52.

The image primary recording means 52 is constituted of VRAM or flash memory or the like, so that when a fundus image is recorded in the image primary recording means 52, it automatically overwrites previously-recorded information.

Thus, a prescribed time period after an image has been recorded in the image primary recording means 52, the timing control circuit 54 outputs a recording command signal S5 from the unit of the eye photographing device to the external recording device 2 (Figure 2 (H)). This causes the control circuit 61 to output to the image secondary recording means 60 an image secondary recording signal S6 as shown in Figure 2 (I), thereby recording the frame of fundus image in the image primary recording means 52 into the image secondary recording means 60. The image secondary recording means 60 is constituted by a hard disk, MO, DVD or printer or the like which can retain a recording even if the power supply is interrupted after the recording operation. Image signals recorded in the image secondary recording means 60 can be reproduced on the monitor 51 of the unit of the eye photographing device via connectors 1c and 2c.

Fundus images recorded in the image primary recording means 52 can also be displayed on the monitor 51. When the images are being displayed or when image signals are being output from the image primary recording means 52 to the image secondary recording means 60, the generation of the strobe flash signal is prohibited, so that, even if the strobe does flash, images cannot be recorded (written) in the image primary recording means 52 because no image primary recording signal S4 is generated. This makes it possible to prevent valuable images being erased by flashing the strobe by mistake or misoperation.

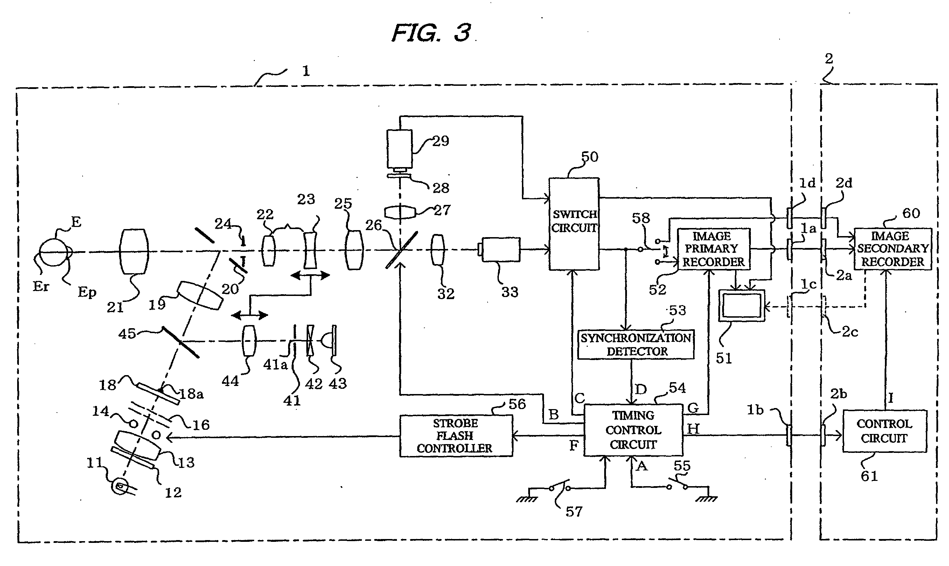

Figure 3 shows another embodiment of the present invention. Parts that are the same as those of Figure 1 have been given identical reference symbols, and further description thereof is omitted.

In the embodiment of Figure 1, recording to an external image secondary recording device is performed automatically in accordance with a recording command signal generated by the timing control circuit 54. In the case of the embodiment of Figure 3, a recording switch 57 is operated to send a recording command signal S5 from the timing control circuit 54 to the control circuit 61 and generate an image secondary recording signal S6, whereby images recorded in the image primary recording means 52 are transferred to, and recorded in, the image secondary recording means 60.

With this arrangement, the examiner can first perform a diagnosis using eye fundus images recorded in the unit of the eye photographing device and then later transfer to the image secondary recording means of the external recording device only those images that need to be permanently preserved in the examiner's judgment. Therefore, this makes it possible to operate the system efficiently by conserving the image recording capacity of the connected recording device.

It is also possible for video signals to be output directly from the visible-light photographing unit 33 to the image secondary recording means 60, not through the image primary recording means 52. A selection switch 58 can be provided for this, as shown in Figure 3. When the selection switch 58 is switched to the lower side in Figure 3, a video signal output from the visible-light photographing unit 33 is first recorded in the image primary recording means 52 as is the same with the embodiment in Figure 1. When the switch 58 is switched to the upper side, a video signal output from the visible-light photographing unit 33 is output directly to the image secondary recording means 60 via connectors 1d and 2d, and is recorded therein without first going to the image primary recording means 52.

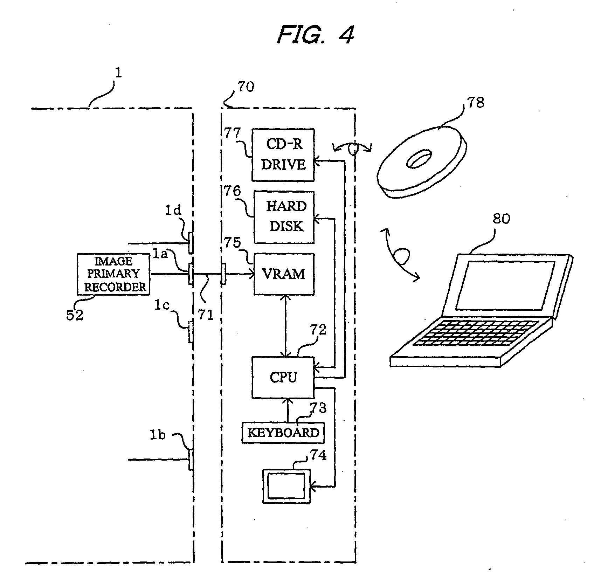

Figure 4 shows still another embodiment of the present invention in which the eye photographing device 1 is connected to a personal computer 70 constituting an external recording device using a connecting cable such as a USB (Universal Serial Bus) cable 71, for example. The personal computer 70 includes a CPU 72, a keyboard 73, a monitor 74, VRAM 75, a hard disk 76 and a CD-R drive 77 that drives a CD-R (recordable compact disc) 78. The USB cable 71 is used to connect the image primary recording means 52 of the eye photographing device 1 to the VRAM 75 of the personal computer 70 via connecter 1a.

In such an arrangement, prescribed keys of the keyboard 73 are operated to transfer images of the fundus of an eye recorded in the image primary recording means 52 to the VRAM 75 under the control of the CPU 72 in order to record them onto the hard disk 76 or the CD-R 78 by the CD-R drive 77. The monitor 74 can be used to observe the recorded images. In cases in which the images are recorded on the CD-R 78, image-reading software is automatically recorded onto the CD-R 78. Thus, even if the CD-R 78 is used in another personal computer 80, the image-reading software recorded on the CD-R 78 makes it possible to view the eye fundus images on the personal computer 80. If a personal computer is equipped with a CD-RW drive with CD-RW (rewritable compact disc) capabilities and the CD-RW is used to record images in the image primary recording means 52, image-reading software that is automatically recorded onto the CD-RW makes it possible to append diagnostic comments or other such information to images viewed on another personal computer 80.

### Industrial Applicability

In accordance with the present invention, photographing is possible independently of the recording preparation settings of externally connected external recording device, as described in the foregoing. For example, eye fundus images can be reliably recorded in an image primary recording means even if an external recording device is not connected to the unit of the eye photographing device, or even if the recording preparation thereof has not been done due to the power supply thereof being off in a state where an external recording device is connected. Photographing errors are therefore eliminated, enabling reliable and smooth photographing of the eye to be examined.

Also, since the timing of the strobe flash in the unit of the photographing device does not have to be controlled via an external recording device, there are few limitations on what recording device is connected, thereby making it possible to readily utilize various types of image recording device. This makes it possible to configure the system in a variety of ways in accordance with a user's situation.

Moreover, there is little possibility of images recorded in the image primary recording means being overwritten by the photographing button being accidentally pressed when the images are being replayed. This allows valuable images to be protected from being erased.

The present invention also makes it possible to perform diagnoses using eye fundus images primary-recorded in the unit of the photographing device, and only those images that, on the basis of the diagnostic results, are deemed necessary to permanently preserve can be transferred to an external image secondary recording means. This makes it possible to operate the system efficiently by conserving the image recording capacity of externally connected recording devices.

Also, the memory of the image primary recording device uses less space and electric power than the hard disk, MO, DVD, printer and the like generally used as the image secondary recording device. This makes it possible to reduce the size of the unit of the photographing device and results in excellent operating stability. This also reduces the risk of recording mistakes caused by malfunction of the recording devices during strobe operation during which patients are burdened.

In addition, recording by the image secondary recording device is controlled from the unit of the photographing device. This allows the image secondary recording device to be located away from the unit, such as outside the examination room, thereby reducing the amount of examination room space required.

In accordance with the present invention, images of an eye to be examined that have been recorded in the image primary recording means can then be recorded on CD-R or CD-RW together with image-reading software. This makes it possible to view the images on a personal computer equipped with a CD-R or CD-RW drive. In the case of CD-RW, information can be appended to the images being viewed, thereby increasing the utility of the photographed images.

## Claims

1. A device for photographing an eye to be examined in which a strobe is made to emit a flash of light to photograph an eye to be examined, comprising:
a means for activating the strobe;
a photographing unit for photographing an eye to be examined illuminated by strobe flash; and
image primary recording means for primarily recording an image of the eye to be examined photographed by the photographing unit,
**characterized in that** an image recorded in the image primary recording means can be recorded in image secondary recording means that is connectable to the image primary recording means.

2. A device for photographing an eye to be examined according to claim 1, **characterized in that** the image primary recording means is provided in a unit of the eye photographing device and the image secondary recording means is provided outside the unit.

3. A device for photographing an eye to be examined according to claim 1 or 2, **characterized in that** the image primary recording means record a prescribed frame of images of the eye to be examined from the photographing unit in synchronization with the strobe flash.

4. A device for photographing an eye to be examined according to one of claims 1 to 3, **characterized in that** a prescribed time after an image of the eye to be examined is recorded in the image primary recording means, the image is automatically recorded in the image secondary recording means.

5. A device for photographing an eye to be examined according to one of claims 1 to 3, **characterized in that** after an image of the eye to be examined is recorded in the image primary recording means, the image is recorded in the image secondary recording means in response to an operation of a recording switch.

6. A device for photographing an eye to be examined according to one of claims 1 to 5, **characterized in that** previously recorded image information is erased and new image information is recorded when it is recorded in the image primary recording means.

7. A device for photographing an eye to be examined according to one of claims 1 to 6, **characterized in that** image information recorded in the image secondary recording means is retained even if electric power from an external source is interrupted.

8. A device for photographing an eye to be examined according to one of claims 1 to 7, characterized that it is provided with means for reproducing and displaying an image recorded in the image primary recording means, wherein an image is inhibited from being recorded in the image primary recording means either during display of an image recorded in the image primary recording means or during recording in the image secondary recording means.

9. A device for photographing an eye to be examined according to one of claims 1 to 8, **characterized in that** a selection switch is operated to record an image of the eye to be examined produced from the photographing unit directly in the image secondary recording means, not through the image primary recording means.

10. A device for photographing an eye to be examined according to claim 5 or.9, **characterized in that** the recording switch or selection switch is provided on the unit of the device for photographing an eye to be examined.

11. A device for photographing an eye to be examined according to one of claims 1 to 3, **characterized in that** an image of the eye to be examined is recorded on CD-R or CD-RW together with image-reading software after the image has been recorded in the image primary recording means.

12. A device for photographing an eye to be examined according to claim 11, **characterized in that** an image of the eye to be examined that is recorded on CD-RW can be viewed by means of image-reading software recorded on the CD-RW and additional information can be appended to the image concerned.

13. A system for photographing an eye to be examined in which a strobe is made to emit a flash of light to photograph an eye to be examined, comprising:
a means for activating the strobe;
a photographing unit for photographing an eye to be examined illuminated by strobe flash;
image primary recording means for primarily recording an image of the eye to be examined photographed by the photographing unit; and
image secondary recording means that is connectable to the image primary recording means;
**characterized in that** an image of the eye to be examined photographed by the photographing unit is recorded in the image primary recording means and then recorded in the image secondary recording means.

14. A system for photographing an eye to be examined according to claim 13, **characterized in that** a prescribed time after an image of the eye to be examined is recorded in the image primary recording means, the image is automatically recorded in the image secondary recording means.

15. A system for photographing an eye to be examined according to claim 13, **characterized in that** after an image of the eye to be examined is recorded in the image primary recording means, the image is recorded in the image secondary recording means in response to an operation of a recording switch.

16. A system for photographing an eye to be examined according to claim 13, **characterized in that** an image of the eye to be examined is recorded on CD-R or CD-RW together with image-reading software after the image has been recorded in the image primary recording means.

17. A system for photographing an eye to be examined according to claim 16, **characterized in that** an image of the eye to be examined that is recorded on CD-RW can be viewed by means of image-reading software recorded on the CD-RW and additional information can be appended to the image concerned.
